# EUROPEAN PATENT SPECIFICATION

(11) **EP 4 091 620 B1**
(45) Date of publication and mention of the grant of the patent: **08.07.2026**
(21) Application number: 22179865.5
(22) Date of filing: 30.01.2019
(51) Int. Cl.: A61K 35/612, A61K 38/01, A23L 33/10, A61K 35/60, A23K 10/22, A23J 3/34, A23K 20/147, A23L 33/18, A61K 31/04, A61K 31/131, A61K 31/198, A61K 31/401, A61K 31/4172, A61K 33/00, A61K 33/06, A61K 33/16, A61K 31/405

(54) **KRILL PROTEIN HYDROLYSATE WITH LOW FLUORIDE AND TRIMETHYLAMIN CONTENT**
KRILL PROTEINHYDROLYSAT MIT NIEDRIGEM FLUORID- UND TRIMETHYLAMINGEHALT
HYDROLYSAT DE PROTÉINES DE KRILL À FAIBLE TENEUR EN FLUOR ET EN TRIMÉTHYLAMINE

(30) Priority: 30.01.2018 US 201862623658 P
(43) Date of publication of application: 23.11.2022
(62) Divisional of application: 19714777.0
(73) Proprietor: Aker BioMarine Understory AS, 1327 Lysaker (NO)
(72) Inventor: ELLEGÅRD, Katrine, Hvid, N-8340 Stamsund (NO); BAGGER, Christian, Lorentz, N-8340 Stamsund (NO); MYHREN, Finn, N-8340 Stamsund (NO); WIKLUND, Erik, Digman, N-8340 Stamsund (NO)
(74) Representative: Acapo Onsagers AS

(56) References cited:
- WO-A1-2010/030193
- WO-A1-2014/184655
- CN-A- 103 960 699
- CN-A- 104 232 717
- CN-A- 106 010 783
- CN-B- 103 238 723
- CN-B- 104 186 911
- CN-B- 104 195 206
- CN-B- 104 263 787
- FR-A1- 2 927 336
- LI LAIHAO ET AL: "STUDIES ON THE EXTRACTION OF FISH PROTEIN CONCENTRATE (FPC) BY SOLVENT EXTRACTION", ASIA PACIFIC FISHING COMISSION, MEETING REPORT, 1 January 1998 (1998-01-01), pages 114 - 119, XP055589785, Retrieved from the Internet <URL:http://www.fao.org/3/a-bm148e.pdf> [retrieved on 20190517]
- J F BERGMANN VERLAG ET AL: "Lebensmittel- Untersuchung und-Forschung A Functional Protein Concentrate (FKPC) from Antarctic Krill (Euphausia superba, Dana 1850)* ** Preparation, Chemical Composition and Functional Properties", Z LEBENSM UNTERS FORSCH, 1 January 1981 (1981-01-01), XP055589787, Retrieved from the Internet <URL:https://link.springer.com/content/pdf/10.1007/BF01127671.pdf> [retrieved on 20190517]

## Description

### FIELD OF THE INVENTION

The present invention provides food products comprising a krill protein hydrolysate as well as use of a krill protein hydrolysate in the preparation of a dietary supplement, nutritional supplement, functional food, food or beverage.

### BACKGROUND OF THE INVENTION

There is an increasing interest in bioactive peptides from marine secondary products, as they offer a great potential for incorporation into functional food and for medical purposes. Bioactive peptides from marine sources have been found to display a wide range of physiological functions including antioxidative, antihypertensive, antimicrobial, immunomodulatory, anticancer and diabetes 2 effects among others. Most of the research has focused on preparation and characterization of hydrolysates for commercial use in health and functional foods.

Krill hydrolysates for use in marine feeds and as human supplements have been described. See, e.g., WO2010030193; WO2013102792; Kolkovski et al., J. World Aqua. Soc., Volume 31, Issue 1 (2000) pp. 81-88. These krill hydrolysates are generally produced from sources that contain high amounts of lipids.

Asia Pacific Fishing Comission, Meeting report, 1 January 1998, pages 114-119 discloses methods for obtaining a fish protein concentrate (FPC) and FPC obtained by said method.

Z Lebensm Unters Forch 172, 393-398 (1981) discloses a method of manufacturing a functional krill protein concentrate which involves alkaline digestion of defatted krill. Products obtained by said method are also being disclosed.

CN 103 960 699 discloses methods for producing a krill peptide-ferrous-zinc-calcium complex. Products and intermediate products obtained by said method are also being disclosed.

CN 104 195 206 discloses a process involving hydrolysis of a defatted krill powder, defluorination, treatment for removing TMAO/TMA/TVN, desalting and spray drying.

CN 104 263 787 discloses a process involving hydrolysis of a defatted krill powder, simulated moving bed (SMB) technology for removing fluoride and ash, and spray drying.

CN 104 232 717 describes a method for reducing fluoride content in water soluble active peptides from krill by using a defluorination agent such as calcium oxide, calcium hydroxide and calcium carbonate. Products obtained by said method are also disclosed herein.

CN 106 010 783 discloses methods for producing krill oil, krill protein peptide powder and chitosan by full utilization of krill powder. The method for preparation of krill protein peptide powder involves enzymatic hydrolysis and the use of calcium oxide for fluoride removal. Products obtained by said methods are also disclosed therein.

CN 104 186 911 describes methods for preparing a protein powder from krill which has thermoreversible gel property. Products obtained by said method are also disclosed therein.

CN 103 238 723 describes a process for obtaining low-fluoride krill protein hydrolysate.

FR 2 927 336 discloses methods of producing a fish protein hydrolysate involving a step of enzymatic hydrolysis. Fish protein hydrolysates and food products comprising said fish protein hydrolysate are also being disclosed.

WO 2014/184655 describes a process for making krill phospholipid protein concentrate with low fluoride content.

### SUMMARY OF THE INVENTION

The present invention provides food products comprising a krill protein hydrolysate as well as use of the krill protein hydrolysate in the preparation of a dietary supplement, nutritional supplement, functional food, food or beverage .

In one embodiment the food product is a nutritional supplement, preferably a nutritional supplement in the form of energy bar, meal replacement bar or a beverage.

In one embodiment the food product is foods selected from the group consisting of beverages, baked goods, puddings, dairy products, confections, snack foods, frozen confections or novelties, prepared frozen meals, candy, snack products, soups, spreads, sauces, salad dressings, prepared meat products, cheese and yogurt. The beverages may be selected from the group consisting of soft drinks, milk and other dairy drinks, and diet drinks; the frozen confections or novelties may be selected from the group consisting of ice cream and milk-shakes; and the snack products may be chips.

In one embodiment the food product is a dietary supplement.

In one embodiment the food product is selected from the group consisting of foods, beverages, functional foods, animal feed rations, powdered protein supplements, meal replacements, beverage powders and protein shake powders.

In one embodiment the food product is an animal feed, such as an animal feed formulated for companion animals or an animal feed formulated for domestic animals.

In one embodiment, the food product is in combination with acceptable excipients and/or carriers, wherein the food product is a formulation for oral consumption. The carrier may be a liquid, a gel, a gelcap, a capsule, a powder, a solid tablet or tea. The acceptable excipient and/or carrier may be selected from the group consisting of vegetable oil, fish oil, krill oil, maltodextrin, calcium carbonate, dicalcium phosphate, tricalcium phosphate, microcrystalline cellulose, dextrose, rice flour, magnesium stearate, stearic acid, croscarmellose sodium, sodium starch glycolate, crospovidone, sucrose, vegetable gums, lactose, methylcellulose, povidone, carboxymethylcellulose, corn starch, and mixtures thereof; in particular calcium carbonate, magnesium stearate, maltodextrin, or mixtures thereof.

In one embodiment, the food product is in combination with flavoring agents or sweeteners and the food product is a formulation for oral administration.

The krill protein hydrolysate is characterized in having a protein content of greater than 85% on a dry weight basis, less than 5% fat on a dry weight basis, a fluoride content of from 0.1 to 200 mg/kg hydrolysate on a dry weight basis, a TMAO (trimethylamine N-oxide) content of from 0.1 to 200 mg N/100 g hydrolysate on a dry weight basis, a TMA (trimethylamine) content of from 0.1 to 200 mg N/100 g hydrolysate on a dry weight basis, a TVN (total volatile nitrogen) content of from 0.1 to 200 mg N/100 g hydrolysate on a dry weight basis, a sodium content of from 0.01 to 4.0g/100g hydrolysate on a dry weight basis, and a calcium content of from 100 to 25,000 mg/kg hydrolysate on a dry weight basis.

The krill protein hydrolysate may also have one or more of the following properties:
g. a lysine content of from 6.30 to 10.30 g lysine/100g total amino acids;
h. a threonine content of from 3.04 to 7.04 g threonine/100g total amino acids;
i. an isoleucine content of from 3.39 to 7.39 g isoleucine/100g total amino acids;
j. a leucine content of from 6.27 to 10.27 g leucine/100g total amino acids;
k. a histidine content of from 0.94 to 3.94 g histidine/100g total amino acids;
1. a phenylalanine content of from 2.76 to 6.76 g phenylalanine/100g total amino acids;
m. a tyrosine content of from 2.39 to 6.39 g tyrosine/100g total amino acids;
n. a valine content of from 3.69 to 7.69 g valine/100g total amino acids;
o. an alanine content of from 3.76 to 7.76 g alanine/100g total amino acids;
p. an arginine content of from 3.90 to 7.90 g arginine/100g total amino acids;
q. an aspartic acid/asparagine content of from 9.68 to 13.68 g aspartic acid and asparagine/100g total amino acids;
r. a glutamic acid/glutamine content of from 11.54 to 17.54 g glutamic acid and glutamine/100g total amino acids;
s. a glycine content of from 2.50 to 6.50 g glycine/100g total amino acids;
t. a proline content of from 1.84 to 5.84 g proline/100g total amino acids;
u. a serine content of from 2.35 to 6.35 g serine/100g total amino acids;
v. a methionine content of from 1.22 to 5.22 g methionine/100g total amino acids;
w. a cysteine and cystine content of from 0.24 to 1.04 g cysteine and cystine/100g total amino acids;
x. a tryptophan content of from 0.76 to 1.76 g tryptophan/100g total amino acids.

In some preferred embodiments, the protein hydrolysate has a fluoride content of from 0.1 to 30 mg/kg on a dry weight basis. In some preferred embodiments, the protein hydrolysate has a TMAO content of from 0.1 to 10 mg N/100 g hydrolysate on a dry weight basis. In some preferred embodiments, the protein hydrolysate has a TMA content of from 0.1 to 30 mg N/100 g hydrolysate on a dry weight basis. In some preferred embodiments, the protein hydrolysate has a TVN content of from 0.1 to 60 mg N/100 g hydrolysate on a dry weight basis.

In some preferred embodiments, the protein hydrolysate further has all of properties g, h, i, j, k, 1, m, n, o, p, q, r, s, t, u, v, w and x.

In some preferred embodiments, the essential amino acid content of the hydrolysate is from 39.36 to 49.36 g essential amino acids/100 g total amino acids. In some preferred embodiments, the branched chain amino acid content of the hydrolysate is from 16.35 to 22.35 g branched chain amino acids/100 g total amino acids. In some preferred embodiments, the sulfur-containing amino acid content of the hydrolysate is from 2.86 to 4.86 g sulfur-containing amino acids/100 g total amino acids.

In some preferred embodiments, the protein hydrolysate is further characterized in having a protein content of greater than 89%, 90%, 91%, 92%, 93%, 94% or 95% on a dry weight basis and a fat content of less than 2% on a dry weight basis. In some preferred embodiments, the protein hydrolysate has a moisture content of less than 3%. In some preferred embodiments, the protein hydrolysate is further characterized in having a neutral, non-fishy taste.

In some preferred embodiments, the protein hydrolysate is further characterized in dispersing into a transparent solution when added to water or other aqueous medium. In some preferred embodiments, the OD 590 of a solution of 4.5 g of the protein hydrolysate dry weight in 100 g water is less than 0.4. In some preferred embodiments, the protein hydrolysate is greater than 80% soluble in water as assayed by dissolving 4.5 g of the protein hydrolysate dry weight in 100 g water. In some preferred embodiments, the protein hydrolysate is greater than 60% soluble in water as assayed by dissolving 4.5 g of the protein hydrolysate dry weight in 100 g water and heating to 85°C for five minutes.

In some preferred embodiments, the krill protein hydrolysates have an astaxanthin content of less than 50 ppm.

The protein hydrolysates as described above may be manufactured by a process comprising: providing a krill meal; milling the krill meal; washing the milled meal with water and/or aqueous citric acid to provide a washed meal; treating the washed meal with a protease to provide a protein hydrolysate; and filtering the hydrolysate by microfiltration with a filter having a pore size of from 0.1 to 10 µm and/or nanofiltration with a filter having a pore size of from 1 to 10 nm to provide a protein hydrolysate and a permeate. The meal may be a solvent-extracted meal. The process may further comprise the step of deactivating the protease prior to filtration. The process may further comprise the step of drying the protein hydrolysate to provide a protein hydrolysate. The krill meal may preferably be a full fat krill meal or a delipidated krill meal. The protease is non-native as compared to the starting krill meal (i.e., the protease does not naturally occur in the krill used to make the meal).

A composition comprising a krill protein hydrolysate concentrate characterized in having a protein content of greater than 85% on a dry weight basis, less than 2% fat on a dry weight basis, and less than 4% moisture is also described herein.

In some preferred embodiments, the krill protein hydrolysate concentrate is further characterized in having a protein content of greater than 89% on a dry weight basis, less than 1% fat on a dry weight basis, and less than 3% moisture. In some preferred embodiments, the krill protein hydrolysate concentrate is further characterized in having an arginine content of from 5 to 7% (g AA/100 g protein). In some preferred embodiments, the krill protein hydrolysate concentrate is further characterized in having a leucine content of from 8 to 10% (g AA/100 g protein). In some preferred embodiments, the krill protein hydrolysate concentrate is further characterized in having a combined branched chain amino acid (i.e., leucine, isoleucine and valine) content of from 17 to 19.5% (g AA/100 g protein). In some preferred embodiments, the krill protein hydrolysate concentrate is further characterized in having a combined content of methionine and cysteine of from 2 to 4% (g AA/100 g protein). In some preferred embodiments, the krill protein hydrolysate concentrate is further characterized in comprising from 50% to 80% peptides of from 2 to 20 amino acids on a w/w basis (peptides of from 2-20 amino acids on length/total weight of free amino acids peptides and polypeptides).

In some preferred embodiments, the krill protein hydrolysate concentrate is further characterized in comprising less than 60 ppm trimethylamine oxide. In some preferred embodiments, the krill protein hydrolysate concentrate is further characterized in comprising less than 50 ppm fluoride. In some preferred embodiments, the krill protein hydrolysate concentrate is further characterized in comprising less than 1% salt. In some preferred embodiments, the krill protein hydrolysate concentrate is further characterized in having a neutral, non-fishy taste. In some preferred embodiments, the krill protein hydrolysate concentrate is further characterized in dispersing into a transparent or clear solution when added to water or other aqueous medium.

Also described herein is processes for producing a krill protein hydrolysate concentrate comprising: providing a solvent-extracted krill meal, washing the solvent extracted krill meal with water and/or citric acid to provide a washed solvent-extracted krill meal, treating the washed solvent extracted krill meal with a protease to provide a krill protein hydrolysate, and filtering the hydrolysate by microfiltration and/or nanofiltration to provide a krill protein hydrolysate concentrate characterized in comprising from 50% to 80% peptides of from 2 to 10 amino acids on a w/w basis (peptides of from 2-10 amino acids on length/total weight of free amino acids peptides and polypeptides) and a permeate.

In some preferred embodiments, the processes further comprise the step of milling the solvent-extracted krill meal prior to washing. In some preferred embodiments, the processes further comprise the step of deactivating the protease prior to filtration. In some preferred embodiments, the processes further comprise the step of drying the krill protein hydrolysate concentrate to provide a granular krill protein hydrolysate concentrate.

In some preferred embodiments, the granular krill protein hydrolysate is characterized in having a protein content of greater than 85% on a dry weight basis, less than 2% fat on a dry weight basis, and less than 4% moisture. In some preferred embodiments, the granular krill protein hydrolysate concentrate is further characterized in having a protein content of greater than 89% on a dry weight basis, less than 1% fat on a dry weight basis, and less than 3% moisture. In some preferred embodiments, the granular krill protein hydrolysate concentrate is further characterized in having an arginine content of from 5 to 7% (g AA/100 g protein). In some preferred embodiments, the granular krill protein hydrolysate concentrate is further characterized in having a leucine content of from 8 to 10% (g AA/100 g protein). In some preferred embodiments, the granular krill protein hydrolysate concentrate is further characterized in having a combined branched chain amino acid content of from 17 to 19.5% (g AA/100 g protein). In some preferred embodiments, the granular krill protein hydrolysate concentrate is further characterized in having a combined content of methionine and cysteine of from 2 to 4% (g AA/100 g protein).

In some preferred embodiments, the granular krill protein hydrolysate concentrate is further characterized in comprising less than 60 ppm trimethylamine oxide. In some preferred embodiments, the granular krill protein hydrolysate concentrate is further characterized in comprising less than 50 ppm fluoride. In some preferred embodiments, the granular krill protein hydrolysate concentrate is further characterized in comprising less than 1% salt. In some preferred embodiments, the granular krill protein hydrolysate concentrate is further characterized in having a neutral, non-fishy taste. In some preferred embodiments, the granular krill protein hydrolysate concentrate is further characterized in dispersing into a clear solution when added to water or other aqueous medium.

A krill protein hydrolysate concentrate made by the processes described above is also described herein.

### DESCRIPTION OF THE DRAWINGS

FIG. 1 is a bar chart showing the amino acid profile of a krill protein hydrolysate.
FIG. 2 is a bar chart showing the peptide size distribution of a krill protein hydrolysate.
FIG. 3 is a flow chart of a process described herein.

### DEFINITIONS

As used herein, the term "hydrolysate" refers to a mixture of amino acids and peptides of different chain length resulting from the enzymatic hydrolysis of a source protein by an added enzyme such as an exogenous protease or protease that is non-native to the source protein.

As used herein, the term "oral delivery vehicle" refers to any means of delivering a pharmaceutical orally, including, but not limited to, capsules, pills, tablets and syrups.

As used herein, the term "food product" refers to any food or feed suitable for consumption by humans, non-ruminant animals, or ruminant animals. The "food product" may be a prepared and packaged food (e.g., mayonnaise, salad dressing, bread, or cheese food) or an animal feed (e.g., extruded and pelleted animal feed or coarse mixed feed). "Prepared food product" means any pre-packaged food approved for human consumption.

As used herein, the term "foodstuff" refers to any substance fit for human or animal consumption.

As used herein, the term "functional food" refers to a food product to which a biologically active supplement has been added.

As used herein, the term "nutritional supplement" refers to a food product formulated as a dietary or nutritional supplement to be used as part of a diet.

As used herein, the term w/w (weight/weight), unless otherwise specified, refers to the amount of a given substance in a composition on a weight basis and is expressed as a percentage of the total composition weight.

As used herein, the term "krill meal" refers to a powder made from krill. Examples of krill meal include, but are not limited to, dried krill meal (e.g., krill meal with a moisture content of the 3 to 15%), delipidated krill meal (e.g., krill meal that has had fat removed by solvent extraction), full-fat krill meal (krill meal that has not been solvent extracted), and subfractions of krill meal.

As used herein, the term "protein," when used in reference to the protein content of a hydrolysate or composition, refers to the content of polypeptides, peptides and amino acids in the hydrolysate or composition. Unless otherwise noted, the protein content is determined by measuring the total nitrogen content, of, for example a hydrolysate, and multiplying by a conversion factor of 6.25. Any suitable method of determining total nitrogen content may be utilized. For example, in some preferred embodiments, the Kjeldahl method is utilized. The content of amino acids in a hydrolysate may be expressed as grams of the amino acid (e.g., as determined by High Performance Liquid Chromatography (HPLC)) per 100 grams of total protein (e.g., as determined by the Kjeldahl method) or as grams of the amino acid per 100 g of total amino acids (e.g., as determined by HPLC of the 20 common amino acids found in proteins).

As used herein, the term "essential amino acids" refers to the nine amino acids that humans cannot synthesize which are histidine, isoleucine, leucine, lysine, methionine, phenylalanine, threonine, tryptophan, and valine.

As used herein, the term "branched chain amino acids" refers to leucine, isoleucine, and valine.

As used herein the term "sulfur-containing amino acids" refers to methionine and cysteine (which may be expressed as cysteine and cystine in the values reported herein).

### DETAILED DESCRIPTION OF THE INVENTION

The present invention provides food products comprising krill protein hydrolysates, and use of the krill protein hydrolysates in the preparation of a dietary supplement, nutritional supplement, functional food, food or beverage. In some preferred embodiments, the krill protein hydrolysates are prepared from whole krill (e.g., fresh or frozen krill), de-shelled krill, or krill meal. In some particularly preferred embodiments, the krill protein hydrolysates are prepared from krill meal, including but not limited to, dried krill meal, delipidated krill meal, full-fat krill meal and fractions of krill meal. The present invention is not limited to the use of any particular krill species. For example, the krill species may be *Euphausia superba* or *Euphausia pacifica.*

During the development of the invention, it was discovered that it is difficult to process krill meal to provide hydrolysates with an acceptable taste and smell along with properties such as a low fluoride content and unique amino acid profile. Previous krill hydrolysates have been described, for example, in WO2010/030193. While that application describes methods for reducing fluoride, the resulting product (believed to marketed as Rimfrost krill powder), contains high amounts of fat and only approximately 55-60% protein. References such as Zhang et al., Fisheries Sci. (2002) 68:672-679 describe krill hydrolysates but provide no evidence of fluoride or TMAO reduction. The quality of the protein produced in Zhang et al. also appears to be low as evidenced by the reported amino acid composition of the hydrolysate.

The present invention addresses problems associated with taste, smell, and fluoride levels and provides a product with an exceptional amino acid composition by utilizing a multistep process that includes washing and/or milling of the krill meal prior to hydrolysis and subsequent filtering steps including nanofiltration. These compositions and processes are exemplified with krill meal but are equally applicable to other meals. As is shown in the examples, the resulting hydrolysate products are characterized by having improved taste and smell (and associated low levels of TMAO (trimethylamine N-oxide), TMA (trimethylamine) and TVN (Total Volatile Nitrogen)) and low fluoride levels while having a unique amino acid profile. The amino acid profile reveals that the krill protein hydrolysates are a complete protein source with sufficient levels of the nine essential amino acids: histidine, isoleucine, leucine, lysine, methionine, phenylalanine, threonine, tryptophan, and valine. As a nonlimiting example, the amino acid score for a krill protein hydrolysate disclosed herein has been calculated to range from 129-159 in comparison to scores of 121 for egg protein and 115 for whey hydrolysate. Complete proteins have a score of 100 or greater on this scale. The amino acid scores are described in detail in WHO (2007): Protein and amino acid requirements in human nutrition. Thus, the krill protein hydrolysates disclosed herein are superior sources of essential amino acids and may be used alone as a complete source of essential amino acids or used to supplement other protein sources which may be lower in essential amino acids.

### 1. Starting materials

In particularly preferred embodiments, the krill protein hydrolysates are prepared from krill meal such as full-fat krill meal or solvent-extracted meal (delipidated krill meal) resulting from extraction of oil from a starting meal, for example, *Euphausia superba* or *Euphausia pacifica* krill meals. Krill meals are preferably made by cooking and drying fresh, most preferably live, krill on board a fishing vessel to provide a krill meal. An exemplary krill meal obtained this process may have an approximate protein content of 70%, approximate fat content of 20% and approximate moisture content of less than 8%.

Krill meal can preferably be made by any standard marine meal process. In general, the krill meal is produced by cooking freshly caught krill at low temperature (approximately 80-85°C) and drying to reduce the moisture content to approximately 5 to 8% and then grinding. In embodiments where the product is intended for human consumption, it is preferable to pack and store the meal under nitrogen without the addition of antioxidants. In some particularly preferred embodiments, the krill meal is a solvent-extracted or delipidated krill meal. The solvent-extracted krill meal may be a residual meal from any type of oil extraction process, for example, extraction with ethanol, methanol, heptane or supercritical solvents such as carbon dioxide with or without an entrainer. In some preferred embodiments, the solvent extracted krill meal is a residual, delipidated krill meal resulting from an extraction process described in PCT/GB2008/001080 or PCT/IB2016/000208.

In some preferred embodiments, krill meal is mixed with a suitable solvent to extract lipids from the meal. An alternative to prior art methods utilizes conditions which preferably extract the maximum amount of lipids from the krill meal at the cost of an increased amount of contaminants in the initial solvent extract. In preferred embodiments, the solvent is an organic protic solvent, however other solvents known for use in extraction of food grade lipids may also be used such as acetone, hexane, etc. Suitable organic protic solvents include, but are not limited to, n-butanol, n-propanol, isopropanol, nitromethane, ethanol, and methanol. In particularly preferred embodiments, the protic solvent is ethanol.

In preferred embodiments, the concentration of the protic solvent used in the initial solvent extraction step is at least 90%, or preferably from about 94% to 98%, more preferably from about 95% to 97%, and is most preferably about 96% (e.g., 96% ethanol or methanol).

In some embodiments, the protic solvent is mixed with the biological starting material at a ratio of protic solvent: biological starting material of about 1:1 to 10:1, preferably about 3:1 to 6:1, more preferably about 4:1 to 5:1, and most preferably about 4.4:1.

In preferred embodiments, the biological starting material is extracted with protic solvent at a temperature of from about 5°C to 65°C, from about 20°C to about 60°C, preferably from about 30°C to 50°C, more preferably from about 30°C to 50°C, and most preferably at about 40°C. In some embodiments, the extraction time (i.e., the amount of time the biological starting material is in contact with the solvent) is from about 10 minutes to about 2 hours, preferably from about 15 minutes to 60 minutes, more preferably from about 20 minutes to about 45 minutes, and most preferably about 30 minutes.

Following the extraction step, a crude krill lipid solution containing the soluble lipids from the krill meal is separated from the solvent/krill meal mixture, for example by decantation and or filtration. The insoluble material, comprising proteins and other useful materials is then dried to recover ethanol. The remaining delipidated krill meal may then be used to make a hydrolysate according to the present invention. In some preferred embodiments, the delipidated krill meal contains less than 15% or 12% wt/wt fat, greater than 65% or 70% wt/wt crude protein and/or less than 5% or 3% w/w moisture.

### 2. Hydrolysate process and compositions

Disclosed herein are processes for producing a krill protein hydrolysate comprising providing a krill meal, washing the krill meal with water and/or citric acid to provide a washed krill meal, and treating the krill meal with a protease to provide a hydrolysate, and filtering the hydrolysate by microfiltration and/or ultrafiltration and/or nanofiltration or diafiltration. In some particularly preferred embodiments, the krill meal is a delipidated krill meal or full fat krill meal. In some preferred embodiments, where the krill meal is a solvent-extracted krill meal, the hydrolysate obtained after filtration is characterized in in comprising from 50% to 80% peptides of from 2 to 10 amino acids on a w/w basis (peptides of from 2-10 amino acids on length/total weight of free amino acids peptides and polypeptides) and a permeate. Additional characteristics of preferred krill protein hydrolysates produced by the process are described in detail below.

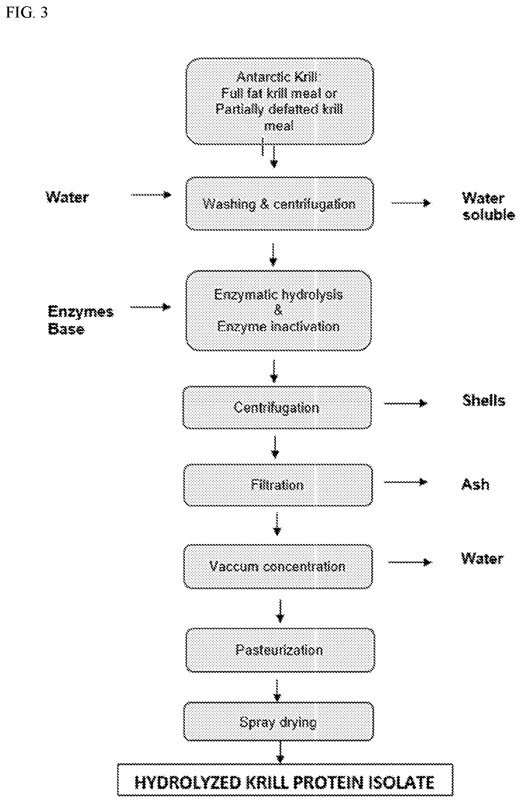

In some preferred embodiments, the processes comprise one or more additional steps, such as those depicted in FIG. 3. The krill meal is milled, preferably wet-milled, prior to washing to reduce particle size. In some preferred embodiments, the milling step reduces particle size of the meal to less than 100 µm and preferably to about 50 µm. The krill meal is then washed with water or another suitable aqueous medium (e.g., an acid solution such as a citric acid solution, a salt solution, or a basic solution). In some embodiments, the washing step comprises a combination of water and acidic washing steps. For example, in some embodiments, the solvent-extracted krill meal is washed first with several volumes of water, followed by a citric acid wash, and then one or more water rinses.

The present invention is not limited to the use of any particular protease or peptidase. In some preferred embodiments, the protease is a serine protease. Suitable serine proteases include subtilisinA and other subtilisinS obtained from, for example, *Bacillus subtilis.* Suitable commercial proteases are marketed by Novozymes and include, but are not limited to, ALCALASE^{™} 2.4 L FG, ALCALASE^{™} 2.5 L, SAVINASE^{™} 12 T, SAVINASE^{™} 16 L, and ESPERASE^{™} 8.0 L. In some preferred embodiments, the processes comprise treating washed and/or milled solvent-extracted krill meal with ALCALASE^{™} at pH of 5 to 9, preferably 7 to 8, and most preferably 7.5 at a temperature of from 30 to 70 °C, more preferably 50 to 60°C, and most preferably at about 60°C, for up to 24 hours (e.g., 30 minutes to 24 hours), for instance from 1-3 hours, about 2 hours, or about 1 hour. Other suitable proteases include, but are not limited to, cysteine proteases, threonine proteases, aspartic proteases, glutamic proteases, metalloproteases and asparagine peptide lysases as are known in the art.

In some preferred embodiments, the processes comprise the step of deactivating the protease prior to filtration. In further preferred embodiments, the processes comprise the step of drying the protein hydrolysate to provide a granular protein hydrolysate. The drying step may be performed by methods known in the art including spray drying. As described in Fig. 3, the process may also preferably comprise additional steps of vacuum concentration, pasteurization and filtration. The process disclosed herein is not limited to the use of any particular filtration steps. In preferred embodiments, a combination of ultrafiltration and nanofiltration is utilized. Ultrafiltration generally utilizes filters, such as membrane filters, with a molecular weight cutoff of approximately 100 kDa. Nanofiltration generally utilizes filters, such as membrane filters, with a pore size of from about 1 to 10 nanometers and, for example, a molecular weight cutoff of approximately 300 Da. In some embodiments, a diafiltration step may be utilized in addition to or in replacement of the nanofiltration step.

The processes disclosed herein produce krill protein hydrolysates with improved organoleptic properties, including improved taste and smell (as evidenced by low TMA, TMAO and TVN levels) as well as low fluoride levels compared to the starting raw material. In some preferred embodiments, the krill protein hydrolysates are characterized in having a protein content of greater than 85% on a dry weight basis, less than 2% fat on a dry weight basis, and less than 4% moisture. In some preferred embodiments, the krill protein hydrolysate is further characterized in having a protein content of greater than 89% on a dry weight basis, less than 1% fat on a dry weight basis, and less than 3% moisture.

The krill hydrolysates disclosed herein are characterized in having a protein content of greater than 85% on a dry weight basis, less than 5% fat on a dry weight basis, and property a to f.
a. a fluoride content of from 0.1 to 200 mg/kg on a dry weight basis, preferably from 0.1 to 30 mg/kg on a dry weight basis; more preferably from 0.1 to 10 mg/kg; even more preferably from 0.1 to 5 mg/kg on a dry weight basis; and most preferably from 0.1 to 3 mg/kg on a dry weight basis; or alternatively, less than 200, 30, 10, 3 or 1 mg/kg F on a dry weight basis;
b. a TMAO content of from 0.1 to 200 mg N/100g hydrolysate on a dry weight basis, preferably from 0.1 to 100 mg N/100g hydrolysate on a dry weight basis; more preferably from 0.1 to 30 mg N/100g hydrolysate on a dry weight basis; even more preferably from 0.1 to 25 mg N/100g hydrolysate on a dry weight basis; and most preferably from 0.1 to 10 mg N/100g hydrolysate on a dry weight basis;
c. a TMA content of from 0.1 to 200 mg N/100g hydrolysate on a dry weight basis, preferably from 0.1 to 100 mg N/100g hydrolysate on a dry weight basis; more preferably from 0.1 to 50 mg N/100g hydrolysate on a dry weight basis; even more preferably from 0.1 to 30 mg N/100g hydrolysate on a dry weight basis; and most preferably from 0.1 to 10 mg N/100g hydrolysate on a dry weight basis;
d. a TVN content of from 0.1 to 200 mg N/100g hydrolysate on a dry weight basis, preferably from 0.1 to 100 mg N/100g hydrolysate on a dry weight basis; more preferably from 0.1 to 60 mg N/100g hydrolysate on a dry weight basis; and most preferably from 0.1 to 20 mg N/100g hydrolysate on a dry weight basis;
e. a sodium content of from 0.01 to 4.0 g/100 g hydrolysate on a dry weight basis, more preferably from 0.1 to 1.0 g/100g hydrolysate on a dry weight basis; and most preferably from 0.01 to 0.2 g/100g hydrolysate on a dry weight basis;
f. a calcium content of from 100 to 25,000 mg/kg hydrolysate on a dry weight basis, more preferably from 10,000 to 25,000 mg/kg hydrolysate on a dry weight basis; and most preferably from 12,000 to 16,000 mg/kg hydrolysate on a dry weight basis.

The krill protein hydrolysate may also have one or more of the following properties:
g. a lysine content of from 6.30 to 10.30 g lysine/100 g total amino acids, preferably from 7.30 to 9.30 g lysine/100 g total amino acids, and more preferably from 7.80 to 8.80 g lysine/100 g total amino acids or alternatively from 6.29 to 10.29 g lysine/100g protein, preferably from 7.29 to 9.29 g lysine/100g protein, and more preferably from 7.79 to 8.79 g lysine/100g protein;
h. a threonine content of from 3.04 to 7.04 grams threonine/100 g total amino acids, preferably from 4.04 to 6.04 g threonine/100 g total amino acids or more preferably from 4.54 to 5.54 g threonine/100 g total amino acids, or alternatively from 3.36 to 7.36 g threonine/100g protein, preferably from 4.36 to 6.36 g threonine/100g protein, more preferably from 4.86 to 5.86 g threonine/100g protein;
i. an isoleucine content of from 3.39 to 7.39 g isoleucine/100 g total amino acids, preferably from 4.39 to 6.39 g isoleucine/100 g total amino acids and more preferably from 4.89 to 5.89 g isoleucine/100 g total amino acids, or alternatively from 3.38 to 7.38 g isoleucine/100g protein, preferably from 4.38 to 6.38 g isoleucine/100g protein, more preferably from 4.88 to 5.88 g isoleucine/100g protein;
j. a leucine content of from 6.27 to 10.27 g leucine/100 g total amino acids, preferably from 7.27 to 9.27 g leucine/100 g total amino acids, or more preferably from 7.77 to 8.77 g leucine/100 g total amino acids, or alternatively from 6.37 to 10.37 g leucine/100g protein, preferably from 7.37 to 9.37 g leucine/100g protein, more preferably from 7.87 to 8.87 g leucine/100g protein;
k. a histidine content of from 0.94 to 3.94 g histidine/100 g total amino acids, preferably from 1.44 to 3.44 g histidine/100 g total amino acids, and more preferably from 1.94 to 2.94 g histidine/100 g total amino acids, or alternatively from 1.09 to 4.09 g histidine/100g protein, preferably from 1.59 to 3.69 g histidine/100g protein, more preferably from 2.09 to 3.09 g histidine/100g protein;
l. a phenylalanine content of from 2.76 to 6.76 g phenylalanine/100 g total amino acids, preferably from 3.76 to 5.76 g phenylalanine/100 g total amino acids, and more preferably from 4.26 to 5.26 g phenylalanine/100 g total amino acids, or alternatively from 3.05 to 7.05 g phenylalanine/100g protein, preferably from 4.05 to 6.05 g phenylalanine/100g protein, more preferably from 4.55 to 5.55 g phenylalanine/100g protein;
m. a tyrosine content of from 2.39 to 6.39 g tyrosine/100 g total amino acids, preferably from 3.39 to 5.39 g tyrosine/100 g total amino acids, and more preferably from 3.89 to 4.89 g tyrosine/100 g total amino acids, or alternatively from 2.46 to 6.46 g tyrosine/100g protein, preferably from 3.46 to 5.46 g tyrosine/100g protein, more preferably from 3.96 to 4.96 g tyrosine/100g protein;
n. a valine content of from 3.69 to 7.69 g valine/100 g total amino acids, preferably from 4.69 to 6.69 g valine/100 g total amino acids, and more preferably from 5.19 to 6.19 g valine/100g total amino acids, or alternatively from 3.88 to 7.88 g valine/100g protein, preferably from 4.88 to 6.88 g valine/100g protein, more preferably from 5.38 to 6.38 g valine/100g protein;
o. an alanine content of from 3.76 to 7.76 g alanine/100 g total amino acids, preferably from 4.76 to 6.76 g alanine/100 g total amino acids, or more preferably from 5.26 to 6.26 g alanine/100 g total amino acids, or alternatively from 3.80 to 7.80 g alanine/100g protein, preferably from 4.80 to 6.80 g alanine/100g protein, more preferably from 5.30 to 6.30 g alanine/100g protein;
p. an arginine content of from 3.90 to 7.90 g arginine/100 g total amino acids, preferably from 4.90 to 6.90 g arginine/100 g total amino acids, or more preferably from 5.40 to 6.40 g arginine/100 g total amino acids, or alternatively from 3.98 to 7.98 g arginine/100g protein, preferably from 4.98 to 6.98 g arginine/100g protein, more preferably from 5.48 to 6.48 g arginine/100g protein;
q. an aspartic acid/asparagine content of from 9.68 to 13.68 g aspartic acid and asparagine/100 g total amino acids, preferably from 10.68 to 12.68 g aspartic acid and asparagine /100 g total amino acids, or more preferably from 11.18 to 12.18 g aspartic acid and asparagine /100 g total amino acids, or alternatively from 10.37 to 14.37 g aspartic acid and asparagine /100g protein, preferably from 11.37 to 13.37 g aspartic acid and asparagine /100g protein, more preferably from 11.87 to 12.87 g aspartic acid and asparagine /100g protein;
r. a glutamic acid/glutamine content of from 11.54 to 17.54 g glutamic acid and glutamine/100 g total amino acids, preferably from 12.54 to 16.54 g glutamic acid and glutamine /100 g total amino acids, or more preferably from 13.54 to 15.54 g glutamic acid and glutamine /100 g total amino acids, or alternatively from 11.66 to 17.66 g glutamic acid and glutamine /100g protein, preferably from 12.66 to 16.66 g glutamic acid and glutamine /100g protein, more preferably from 13.66 to 15.66 g glutamic acid and glutamine /100g protein;
s. a glycine content of from 2.50 to 6.50 glycine/100 g total amino acids, preferably from 3.50 to 5.50 g glycine/100 g total amino acids, and more preferably from 4.00 to 5.00 g glycine/100 g total amino acids or alternatively from 2.70 to 6.70 g glycine/100g protein, preferably from 3.70 to 5.70 g glycine/100g protein, more preferably from 4.20 to 5.20 g glycine/100g protein;
t. a proline content of from 1.84 to 5.84 g proline/100 g total amino acids, preferably from 2.84 to 4.84 g proline/100 g total amino acids, or more preferably from 3.34 to 4.34 g proline/100 g total amino acids, or alternatively from 2.27 to 6.27 g proline/100g protein, preferably from 3.27 to 5.27 g proline/100g protein, more preferably from 3.77 to 4.77 g proline/100g protein;
u. a serine content of from 2.35 to 6.35 g serine/100 a total amino acid, preferably from 3.35 to 5.35 g serine/100 g total amino acids, and more preferably from 3.85 to 4.85 g serine/100 g total amino acids, or alternatively from 2.64 to 6.64 g serine/100g protein, preferably from 3.64 to 5.64 g serine/100g protein, more preferably from 4.14 to 5.14 g serine/100g protein;
v. a methionine content of from 1.22 to 5.22 g methionine/100 g total amino acids, preferably from 2.22 to 4.22 g methionine/100 g total amino acids, or more preferably 2.72 to 3.72 g methionine/100 g total amino acids, or alternatively from 1.69 to 5.69 g methionine/100g protein, preferably from 2.69 to 4.69 g methionine/100g protein, more preferably from 3.19 to 4.19 g methionine/100g protein;
w. a cysteine/cystine content of from 0.24 to 1.04 g cysteine and cystine/100 g total amino acids, preferably from 0.44 to 0.84 g cysteine and cystine /100 g total amino acids, or more preferably from 0.54 to 0.74 cysteine and cystine/100 g total amino acids, or alternatively from 0.249 to 1.049 g cysteine and cystine/100g protein, preferably from 0.449 to 0.849 g cysteine and cystine/100g protein, more preferably from 0.549 to 0.749 g cysteine and cystine/100g protein;
x. a tryptophan content of from 0.76 to 1.76 g tryptophan/100 g total amino acids, preferably from 0.96 to 1.56 g tryptophan/100 g total amino acids, or more preferably from 1.06 to 1.46 g tryptophan/100 g total amino acids, or alternatively from 0.8 to 1.8 g tryptophan/100g protein, preferably from 1.0 to 1.6 g tryptophan/100g protein, and, more preferably from 1.1 to 1.5 g tryptophan/100g protein.

As can be seen, the ranges for total amino acids content are provided in grams of the specified amino acid per 100 grams of total amino acids (of the 20 amino acids found in proteins) as measured by HPLC or alternatively as grams of amino acid (measured by HPLC) per 100 grams of total protein (e.g., as measured by the Kjeldahl method).

In some even more preferred embodiments, the hydrolysates described above have a protein content of greater than 90%, 91%, 92%, 93%, 94% or 95% on a dry weight basis and/or a fat content of less than 3%, 2% or 1% on a dry weight basis.

In some preferred embodiments, the hydrolysates further have a fluoride content of from 0.1 to 30 ppm, and/or a TMAO content of from 0.1 to 10 ppm; and/or a TMA content of from 0.1 to 30 ppm; and/or a TVN content of from 0.1 to 60 ppm.

In some preferred embodiments, the hydrolysates are further characterized in having an essential amino acid content of from 39.36 to 49.36 g essential amino acids/100 g total amino acids, preferably from 41.36 to 47.36 g essential amino acids/100 g total amino acids, more preferably from 42.36 to 46.36 g essential amino acids/100 g total amino acids, and most preferably from 43.36 to 45.36 g essential amino acids/100 g total amino acids.

In some preferred embodiments, the hydrolysates are further characterized in having a branched chain amino acid content of from 16.35 to 22.35 g branched chain amino acids/100 g total amino acids, 17.35 to 21.35 g branched chain amino acids/100 g total amino acids, and more preferably from 18.35 to 20.35 g branched chain amino acids/100 g total amino acids.

In some preferred embodiments, the hydrolysates are further characterized in have a sulfur-containing amino acid content of from 2.86 to 4.86 g sulfur-containing amino acids/100 g total amino acids, preferably from 3.16 to 4.56 g sulfur-containing amino acids/100 g total amino acids, and more preferably from 3.46 to 4.26 g sulfur-containing amino acids/100 g total amino acids.

In some preferred embodiments, the protein hydrolysates are further characterized in having an arginine content of from 5 to 7% (g Amino Acid ("AA")/100 g protein). In some preferred embodiments, the protein hydrolysates are further characterized in having a leucine content of from 7 to 10% (g AA/100 g protein). In some preferred embodiments, the protein hydrolysates are further characterized in having a combined branched chain amino acid content of from 17 to 19.5% (g AA/100 g protein). In some preferred embodiments, the protein hydrolysates are further characterized in having a combined content of methionine and cysteine of from 2 to 4% (g AA/100 g protein). In some preferred embodiments, the protein hydrolysates are further characterized in having a content of tryptophan of from 0.1% to 3% (g AA/100 g protein), more preferably from 0.2% to 2% (g AA/100 g protein). In some preferred embodiments, the protein hydrolysates are further characterized in comprising from 50% to 80% peptides of from 2 to 10 amino acids on a w/w basis (peptides of from 2-10 amino acids on length/total weight of free amino acids peptides and polypeptides). In some preferred embodiments, the protein hydrolysates are further characterized in comprising from 50% to 80% peptides of from 2 to 20 amino acids on a w/w basis (peptides of from 2-20 amino acids on length/total weight of free amino acids peptides and polypeptides). In some preferred embodiments, the protein hydrolysates are further characterized in comprising from 50% to 80% peptides of from 2 to 15 amino acids on a w/w basis (peptides of from 2-15 amino acids on length/total weight of free amino acids peptides and polypeptides). In some preferred embodiments, the protein hydrolysates are is further characterized in comprising from 50% to 80% peptides of from 2 to 20 amino acids on a w/w basis (peptides of from 2-10 amino acids on length/total weight of free amino acids peptides and polypeptides).
In some preferred embodiments, the krill protein hydrolysates are further characterized in comprising less than 60 ppm trimethylamine oxide. In some preferred embodiments, the krill protein hydrolysates are further characterized in comprising less than 50 ppm fluoride. In some preferred embodiments, the krill protein hydrolysates are further characterized in comprising less than 4% and most preferably less than 1% sodium. In some preferred embodiments, the krill protein hydrolysates are further characterized in having a neutral, non-fishy taste.

In some preferred embodiments, the krill protein hydrolysates are further characterized in dispersing into a clear solution when added to water or other aqueous medium. For example, in some embodiments, when 4.5 grams of a hydrolysate disclosed herein on a dry weight basis is dissolved into 100 g water at pH 3-9 (e.g., pH 6) the optical density at 590 nm (OD 590) is less than 0.4, preferably less than 0.3. In some embodiments, the hydrolysates preferably have from 0.1 to 50 ppm, 0.1 to 10 ppm or 0.1 to 5 ppm astaxanthin and more preferably less than 50 ppm, 10 ppm or 5ppm astaxanthin.

In some embodiments, the solubility of the krill protein hydrolysates may be assayed by dissolving 4.5 gram of the hydrolysate on a dry weight basis in 100 grams of water at 20 - 25°C (e.g., 23°C), stirring, centrifuging the solution at 1500 g for 5 minutes, and measuring the dry weight of the material in solution after centrifugation. In some preferred embodiments, the solubility of the hydrolysate is more than 80%, 90%, 91%, 92%, 92%, 94% at pH 3-7 (e.g., pH 3.5) as measured by this assay and more than 75%, 80% or 90% at pH 9. In some embodiments, solubility following heat treatment at 85°C for 5 minutes is greater than 60% as measured by the assay at pH 3-9 (e.g., pH 6).

### 3. Hydrolysate products

The hydrolysates disclosed herein may be incorporated into a variety of products. Accordingly the present invention provides foods, beverages, functional foods, animal feed rations, powdered protein supplements, meal replacements, beverage powders, protein shake powders and the like comprising a krill protein hydrolysate disclosed herein.

The hydrolysates are preferably administered orally. Accordingly, in some embodiments, the hydrolysates disclosed herein (such as those described in the preceding sections) are formulated with acceptable excipients and/or carriers for oral consumption. The actual form of the carrier, and thus, the composition itself, is not critical. The carrier may be a liquid, gel, gelcap, capsule, powder, solid tablet (coated or non-coated), tea, or the like. Oral delivery vehicles are preferably in the form of a tablet or capsule. Suitable excipient and/or carriers include vegetable oil, fish oil, krill oil, maltodextrin, calcium carbonate, dicalcium phosphate, tricalcium phosphate, microcrystalline cellulose, dextrose, rice flour, magnesium stearate, stearic acid, croscarmellose sodium, sodium starch glycolate, crospovidone, sucrose, vegetable gums, lactose, methylcellulose, povidone, carboxymethylcellulose, corn starch, and the like (including mixtures thereof). Preferred carriers include calcium carbonate, magnesium stearate, maltodextrin, and mixtures thereof. The various ingredients and the excipient and/or carrier are mixed and formed into the desired form using conventional techniques. The tablet or capsule of the present invention may be coated with an enteric coating that dissolves at a pH of about 6.0 to 7.0. A suitable enteric coating that dissolves in the small intestine but not in the stomach is cellulose acetate phthalate. Further details on techniques for formulation for and administration may be found in the latest edition of Remington's Pharmaceutical Sciences (Maack Publishing Co., Easton, PA).

In some embodiments, the hydrolysates are formulated for oral administration with flavoring agents or sweeteners. Examples of useful flavoring include, but are not limited to, pure anise extract, imitation banana extract, imitation cherry extract, chocolate extract, pure lemon extract, pure orange extract, pure peppermint extract, imitation pineapple extract, imitation rum extract, imitation strawberry extract, or pure vanilla extract; or volatile oils, such as balm oil, bay oil, bergamot oil, cedarwood oil, walnut oil, cherry oil, cinnamon oil, clove oil, or peppermint oil; peanut butter, chocolate flavoring, vanilla cookie crumb, butterscotch or toffee. In one embodiment, the dietary supplement contains cocoa or chocolate. Emulsifiers may be added for stability of the final product. Examples of suitable emulsifiers include, but are not limited to, lecithin (e.g., from egg or soy), and/or mono- and di-glycerides. Other emulsifiers are readily apparent to the skilled artisan and selection of suitable emulsifier(s) will depend, in part, upon the formulation and final product. In addition to the carbohydrates described above, the nutritional supplement can contain natural or artificial (preferably low calorie) sweeteners, e.g., saccharides, cyclamates, aspartamine, aspartame, acesulfame K, and/or sorbitol.

The dietary supplement may comprise one or more inert ingredients, especially if it is desirable to limit the number of calories added to the diet by the dietary supplement. For example, the dietary supplement of the present invention may also contain optional ingredients including, for example, herbs, vitamins, minerals, enhancers, colorants, sweeteners, flavorants, inert ingredients, and the like. For example, the dietary supplement of the present invention may contain one or more of the following: ascorbates (ascorbic acid, mineral ascorbate salts, rose hips, acerola, and the like), dehydroepiandosterone (DHEA), green tea (polyphenols), inositol, kelp, dulse, flavonoids, maltodextrin, nettles, niacin, niacinamide, rosemary, selenium, silica (silicon dioxide, silica gel, horsetail, shavegrass, and the like), spirulina, zinc, and the like. Such optional ingredients may be either naturally occurring or concentrated forms.

In some embodiments, the dietary supplements further comprise vitamins and minerals including, but not limited to, calcium phosphate or acetate, tribasic; potassium phosphate, dibasic; magnesium sulfate or oxide; salt (sodium chloride); potassium chloride or acetate; ascorbic acid; ferric orthophosphate; niacinamide; zinc sulfate or oxide; calcium pantothenate; copper gluconate; riboflavin; beta-carotene; pyridoxine hydrochloride; thiamin mononitrate; folic acid; biotin; chromium chloride or picolonate; potassium iodide; sodium selenate; sodium molybdate; phylloquinone; vitamin D₃; cyanocobalamin; sodium selenite; copper sulfate; vitamin A; vitamin C; inositol; potassium iodide. Suitable dosages for vitamins and minerals may be obtained, for example, by consulting the U.S. RDA guidelines.

In other embodiments, the present invention provides nutritional supplements (e.g., energy bars or meal replacement bars or beverages) comprising of the hydrolysates disclosed herein. The nutritional supplement may serve as meal or snack replacement and generally provide nutrient calories. Preferably, the nutritional supplements provide carbohydrates, proteins, and fats in balanced amounts. The nutritional supplement can further comprise carbohydrate, simple, medium chain length, or polysaccharides, or a combination thereof. A simple sugar can be chosen for desirable organoleptic properties. Uncooked cornstarch is one example of a complex carbohydrate. If it is desired that it should maintain its high molecular weight structure, it should be included only in food formulations or portions thereof which are not cooked or heat processed since the heat will break down the complex carbohydrate into simple carbohydrates, wherein simple carbohydrates are mono- or disaccharides. The nutritional supplement contains, in one embodiment, combinations of sources of carbohydrate of three levels of chain length (simple, medium and complex; e.g., sucrose, maltodextrins, and uncooked cornstarch).

In still further embodiments, the present invention provides food products, prepared food products, or other foodstuffs (e.g., functional foods) comprising the hydrolysates disclosed herein. In preferred embodiments, the foods comprise an effective amount of the components as described above. For example, in some embodiments, beverages and solid or semi-solid foods comprising the hydrolysates are provided. These forms can include, but are not limited to, beverages (e.g., soft drinks, milk and other dairy drinks, and diet drinks), baked goods, puddings, dairy products, confections, snack foods, or frozen confections or novelties (e.g., ice cream, milk shakes), prepared frozen meals, candy, snack products (e.g., chips), soups, spreads, sauces, salad dressings, prepared meat products, cheese, yogurt and any other fat or oil containing foods, and food ingredients (e.g., wheat flour).

In some preferred embodiments, the hydrolysates are incorporated into chewable matrices. Preferred chewable matrices jelly candies and gelatin-based gummi candy. Exemplary gummi candies include gummi bears, gummi worms, gummi frogs, gummi hamburgers, gummi cherries, gummi soda bottles, gummi sharks, gummi army men, gummi hippopotami, gummi lobsters, gummi watermelons, gummi octopuses, gummi apples, gummi peaches, and gummi oranges. The terms "gummi" and "gummy" are used interchangeably herein.

As will be appreciated, the hydrolysates disclosed herein may be delivered as dietary supplements, nutritional supplements, or functional foods or be incorporated into foods or beverages. Accordingly, methods for preparing a dietary supplement, nutritional supplement, functional food, food or beverage comprising mixing a protein hydrolysate as described above with one or more additional components to form the desired product (i.e., dietary supplement, nutritional supplement, functional food, food or beverage) are described herein. The additional components for these products are described in detail above. Further, the present invention provides for the use of the krill protein hydrolysates described herein as an ingredient in the preparation of a dietary supplement, nutritional supplement, functional food, food or beverage.

In some preferred embodiments, the hydrolysates are incorporated into animal feeds and rations. Animals feeds according to the present invention may be formulated for companion animals such as cats and dogs as well as domestic and wild animals including domestic poultry, hogs, cattle, sheep, rabbits, and birds, fish such as trout, salmon, catfish and tilapia, shrimp, and other species produced via aquaculture. Many different feed rations may be formulated for animals from many different feed ingredients. Rations are generally formulated to provide nutrients in accordance with National Research Council standards. The feedstuffs used in the ration (in addition to the hydrolysates) can be chosen according to market price and availability. Thus, some components of the ration may change over time. In the feeds of the present invention, the ration will contain a hydrolysate described herein as part of the protein component, but other components may vary over time based on the price of the component. For discussions on feed ration formulation, actual rations and NRC guidelines, see Church, Livestock Feeds and Feeding, O&B Books, Inc., Corvallis, Oreg. (1984) and Feeds and Nutrition Digest, Ensminger, Oldfield and Heineman eds., Ensminger Publishing Corporation, Clovis, Calif. (1990).

The animal feed rations of the present invention may be characterized according to NRC requirements. NRC requirements may be found in Church, Livestock Feeds and Feeding, O&B Books, Inc., Corvallis, Oreg. (1984), or other nutritional standards. Animal rations are traditionally balanced using the protein and energy requirements, and then adjusted if needed to meet the other requirements. The animal rations of the present invention will contain a hydrolysate according to the present invention, for example from 0.5% to 30% wt/wt, 0.5% to 20% wt/wt. 0.5% to 10% wt/wt or 0.5% to 5% wt/wt hydrolysate in addition to other feed materials necessary to balance the feed to meet the NRC requirements for the different stages of growth and maintenance. The relative amounts of protein and energy are adjusted to reflect Nutritional Standards requirements. The amounts of feed components will vary with the stage of animal fed. A growing ration for young animals will have higher protein levels, while a finishing ration for finishing animals for market will have higher energy values which are supplied by carbohydrates. In some feeding situations, care must be taken to provide the appropriate amino acids as well as overall protein content. In most animal diets, energy requirements are met by starches in cereal grains. Energy requirements may also be met by addition of fat to the ration. In the present invention, the CFAP provides part of the energy requirement.

Other ingredients may be added to the feed ration. These ingredients include, but are not limited to, mineral supplements such as calcium, phosphorus, salt, selenium and zinc; vitamin supplements such as Vitamins A, B, D, E, and K; amino acid supplements such as lysine; coccidiostats, or growth promoters such as bacitracin or virginamycin; and other active drugs such as chlortetracycline, sulfathiozole, and penicillin. For vitamin, mineral and antibiotic supplement formulation see Church, Livestock Feeds and Feeding, O&B Books, Inc., Corvallis, Oreg. (1984).

In a preferred embodiment, the hydrolysate is incorporated into a pelleted feed for administration to domestic animals. Pelleted feed is created by first mixing feed components and then compacting and extruding the feed components through a die with heat and pressure. The feed is pelleted by methods known in the art, which are described in MacBain, Pelleting Animal Feed, American Feed Manufacturers Association, Arlington, Va. (1974), incorporated herein by reference. When incorporating added fat into pelleted feed, caution is needed in order to avoid making mealy pellets. Generally, only about 2% of the fat is added during pelleting, with the rest added after the pellets have cooled.

### Example 1

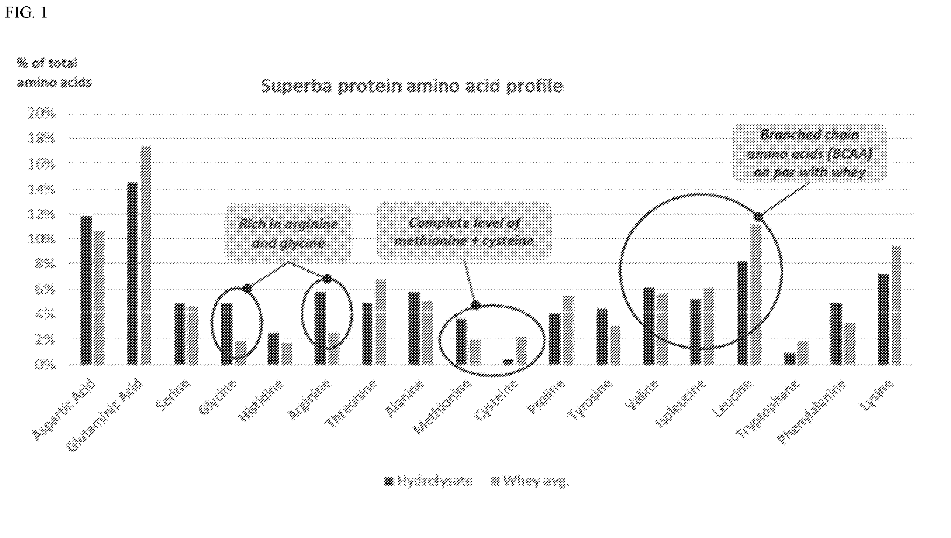

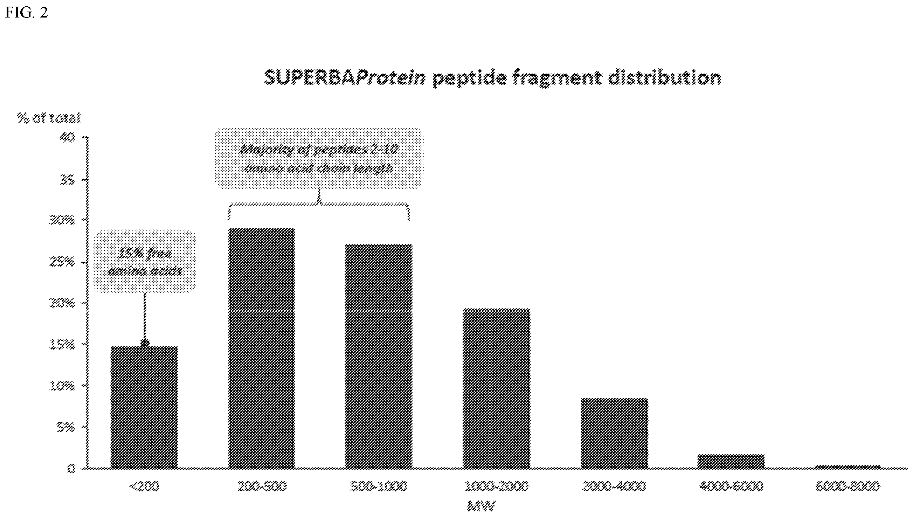

Solvent-extracted krill meal produced according to the process described in PCT/IB2016/000208 is milled in a pin meal at 16,000 RPM to a mean particle size of 50 µm. The milled protein composition is then washed with citric acid (65°C, 9 min., 0.19M, pH 4) followed by a 3 hot water washes at 65°C for 9 min. The washed protein composition is then treated with ALCALASE for 2.5 hours at pH 7.5 and 52.5°C and then washed with hot water for 9 min. at 65°C. The resulting hydrolysate is then micro- and nanofiltered (1.25 L/h/m2/bar, < 5 bar pressure) to provide a concentrate. The concentrate is then granulated by spray drying (agglomeration, three stage drying, inlet 182°C, outlet 82.5°C). The hydrolysate concentrate has the properties listed in Tables 1-3 and in Figs. 1 and 2.

**Table 1.**

| **Parameter:** | **Limits:** | **Method of analysis:** |
|---|---|---|
| **Physical properties** | | |
| Flavor/Odor | Bland | Organoleptic |
| Appearance | Off-white powder | Visual |

| **Chemical analysis** | | |
|---|---|---|
| Moisture | Max 3.0 % | Drying |
| Protein, As is | Min 87.5 % | Kjeldahl |
| Protein, Dry basis | Min 90.0 % | Kjeldahl |
| Total Fat | Max 1.0 % | Soxhlet |
| Ash | Max 6.0 % | Gravimetric |
| Carbohydrates | Max 5.0 % | HPLC |
| Sodium (Na) | Max 1.0 % | ICP-MS |

| **Microbiology** | | |
|---|---|---|
| Total Plate Count | Max 1,000 CFU/g | USP |
| Salmonella | Negative / 25 g | USP |
| Enterobacteriacea | Negative / 25 g | USP |
| E. coli | Negative / 10 g | USP |
| Yeast and Mold | Max 100 CFU/g | USP |

**Table 2.**

| **Typical Quantity per 100 g Product** | | |
|---|---|---|
| **Calories (kcal)** | | **373** |
| | - From total fat | 9 |
| | - From Carbohydrate | 8 |
| | - From Protein | 356 |
| | | |
| **Protein** | | **89 g** |
| **Moisture** | | **2 g** |
| **Ash** | | **4 g** |
| **Crude Fat** | | **1 g** |
| **Total Carbohydrate** | | **2 g** |

**Table 3.**

| **Amino Acid content** | **Typical g AA/100g product** | **Typical g AA/100g protein** |
|---|---|---|
| Alanine | 4.0 | 4.3 |
| Arginine | 5.4 | 5.8 |
| Aspartic Acid | 7.6 | 8.2 |
| Cysteine | 0.4 | 0.5 |
| Glutamic Acid | 9.8 | 10.6 |
| Glycine | 5.3 | 5.7 |
| Histidine | 2.8 | 3.1 |
| Isoleucine | 4.1 | 4.5 |
| Leucine | 8.5 | 9.3 |
| Lysine | 5.9 | 6.4 |
| Methionine | 2.4 | 2.6 |
| Phenylalanine | 5.8 | 6.3 |
| Proline | 2.7 | 3.0 |
| Serine | 3.2 | 3.4 |
| Threonine | 3.6 | 3.9 |
| Tyrosine | 3.3 | 3.5 |
| Tryptophan | Not determined | Not determined |
| Valine | 4.2 | 4.6 |

### Example 2

Two processes for making a krill hydrolysate were compared. Except as noted, the process in Fig. 3 was utilized. In the first process, there was no wet milling and no ultrafiltration step. The hydrolysis time was 1 hour. The resulting hydrolysate is referred to as "Batch A." A nanofiltration step was utilized after hydrolysis. The second process utilized wet milling prior to hydrolysis and both ultrafiltration and nanofiltration after hydrolysis. The hydrolysis time was 14.5 hours. The resulting hydrolysate is referred to as "Batch B." A comparison of selected values for Batch A and Batch B is provided in Table 4. Table 5 provides the amino acid composition of Batch B and also average amino acid content across multiple krill hydrolysate batches made by the process of the invention. In the third column of Table 5, the amino acid content is expressed as grams amino acid per 100 grams protein. The grams of amino acid in 100 g hydrolysate was determined by high performance liquid chromatography (HPLC). The protein content was determined by the Kjeldahl method. In the fourth column of Table 5, amino acid content is listed for combined batches made from different meals, including delipidated and full fat krill meals. In column 4, the amino acid content is listed in grams of the indicated amino acid per 100 grams total amino acids (as measured by HPLC). As can be seen, the Batch B hydrolysate has substantially improved values for fluoride (F) content, TMAO, TMA and TVN content. A subsequent batch ("Batch C") was found to have a F content of 1.09 mg/kg on a dry weight basis as well as low values for TMA, TMAO, and TVN. The hydrolysates have excellent amino acid composition, especially with respect to the content and composition of the essential and branched chain amino acids.

**Table 4. Comparison of Batch A hydrolysate and Batch B hydrolysate.**

| **Property** | **BATCH A** | **BATCH B** |
|---|---|---|
| F (mg/kg dry weight basis) | 277 | 8.39 |
| TMAO (mg N/100g, dry weight basis) | 60 | 3 |
| TMA (mg N/100g, dry weight basis) | | <30 |
| TVN (mg N/100g, dry weight basis) | | 37.7 |
| Protein% as is | 87.3 | 92.1 |
| Fat% as is | 0.175 | <0.4 |
| Water content% as is | 4.6 | 1.4 |
| Water activity | | 0.14 |

**Table 5. Amino acid composition of Batch B hydrolysate and average amino acid content across batches.**

| | **Batch B** | | **Average across batches** |
|---|---|---|---|
| **Amino Acid** | **g/100g hydrolysate** | **g/100g protein** | **g/100 g total amino acids** |
| Lysine | 7.64 | 8.29 | 8.30 |
| Threonine | 4.94 | 5.36 | 5.04 |
| Isoleucine | 4.96 | 5.38 | 5.39 |
| Leucine | 7.71 | 8.37 | 8.27 |
| Histidine | 2.39 | 2.59 | 2.44 |
| Phenylalanine | 4.66 | 5.05 | 4.76 |
| Tyrosine | 4.11 | 4.46 | 4.39 |
| Valine | 5.42 | 5.88 | 5.69 |
| Alanine | 5.35 | 5.80 | 5.76 |
| Arginine | 5.51 | 5.98 | 5.90 |
| Aspartic acid + Asparagine | 11.4 | 12.37 | 11.68 |
| Glutamic acid + Glutamine | 13.5 | 14.66 | 14.54 |
| Glycine | 4.33 | 4.70 | 4.50 |
| Proline | 3.93 | 4.27 | 3.84 |
| Serine | 4.27 | 4.64 | 4.35 |
| Methionine | 3.40 | 3.69 | 3.22 |
| Cysteine + Cystine | 0.598 | 0.649 | 0.64 |
| Tryptophan | 1.2 | 1.30 | 1.26 |
| Sum | 95.47 | | |

## Claims

1. A food product comprising a krill protein hydrolysate, the krill protein hydrolysate comprising:
- a protein content of greater than 85% on a dry weight basis;
- less than 5% fat on a dry weight basis;
- a fluoride content of from 0.1 to 200 mg/kg hydrolysate on a dry weight basis;
- a TMAO (trimethylamine N-oxide) content of from 0.1 to 200 mg N/100 g hydrolysate on a dry weight basis;
- a TMA (trimethylamine) content of from 0.1 to 200 mg N/100 g hydrolysate on a dry weight basis;
- a TVN (total volatile nitrogen) content of from 0.1 to 200 mg N/100 g hydrolysate on a dry weight basis;
- a sodium content of from 0.01 to 4.0 g/100 g hydrolysate on a dry weight basis; and
- a calcium content of from 100 to 25,000 mg / kg hydrolysate on a dry weight basis.

2. The food product of claim 1, wherein the food product is a nutritional supplement, preferably a nutritional supplement in the form of energy bar, meal replacement bar or a beverage.

3. The food product of claim 1, wherein the food product is foods selected from the group consisting of beverages, baked goods, puddings, dairy products, confections, snack foods, frozen confections or novelties, prepared frozen meals, candy, snack products, soups, spreads, sauces, salad dressings, prepared meat products, cheese and yogurt.

4. The food product of claim 3, wherein
- the beverages are selected from the group consisting of soft drinks, milk and other dairy drinks, and diet drinks;
- the frozen confections or novelties are selected from the group consisting of ice cream and milk-shake; and
- the snack products are chips;

5. The food product of claim 1, wherein the food product is a dietary supplement.

6. The food product of claim 1, wherein the food product is selected from the group consisting of foods, beverages, functional foods, animal feed rations, powdered protein supplements, meal replacements, beverage powders and protein shake powders.

7. The food product of claim 1, wherein the food product is an animal feed, such as an animal feed formulated for companion animals or an animal feed formulated for domestic animals.

8. The food product of claim 1, in combination with acceptable excipients and/or carriers, wherein the food product is a formulation for oral consumption.

9. The food product of claim 8, wherein the carrier is a liquid, a gel, a gelcap, a capsule, a powder, a solid tablet or tea.

10. The food product of claim 8, wherein the acceptable excipient and/or carrier is selected from the group consisting of vegetable oil, fish oil, krill oil, maltodextrin, calcium carbonate, dicalcium phosphate, tricalcium phosphate, microcrystalline cellulose, dextrose, rice flour, magnesium stearate, stearic acid, croscarmellose sodium, sodium starch glycolate, crospovidone, sucrose, vegetable gums, lactose, methylcellulose, povidone, carboxymethylcellulose, corn starch, and mixtures thereof; in particular calcium carbonate, magnesium stearate, maltodextrin, or mixtures thereof.

11. The food product of claim 1, in combination with flavoring agents or sweeteners, wherein the food product is a formulation for oral administration,

12. Use of a krill protein hydrolysate in the preparation of a dietary supplement, nutritional supplement, functional food, food or beverage; the krill protein hydrolysate comprising:
- a protein content of greater than 85% on a dry weight basis;
- less than 5% fat on a dry weight basis;
- a fluoride content of from 0.1 to 200 mg/kg hydrolysate on a dry weight basis;
- a TMAO (trimethylamine N-oxide) content of from 0.1 to 200 mg N/100 g hydrolysate on a dry weight basis;
- a TMA (trimethylamine) content of from 0.1 to 200 mg N/100 g hydrolysate on a dry weight basis;
- a TVN (total volatile nitrogen) content of from 0.1 to 200 mg N/100 g hydrolysate on a dry weight basis;
- a sodium content of from 0.01 to 4.0 g/100 g hydrolysate on a dry weight basis; and
- a calcium content of from 100 to 25,000 mg / kg hydrolysate on a dry weight basis.

13. The food product according to any one of claims 1-11 or the use according to claim 12, wherein the krill protein hydrolysate has a fluoride content of from 0.1 to 30 mg/kg on a dry weight basis.

14. The food product according to any one of claims 1-11 or the use according to claim 12, wherein the krill protein hydrolysate is further **characterized in** having a protein content of greater than 89% on a dry weight basis and a fat content of less than 2% on a dry weight basis.

15. The food product according to any one of claims 1-11 or the use according to claim 12, wherein the krill protein hydrolysate is **characterized in** having a protein content of greater than 90%, 91%, 92%, 93%, 94% or 95% on a dry weight basis and/or a fat content of less than 3%, 2% or 1% on a dry weight basis.

## Patentansprüche

1. Nahrungsmittelprodukt, umfassend ein Krillproteinhydrolysat, wobei das Krillproteinhydrolysat Folgendes umfasst:
- einen Proteingehalt von mehr als 85 % auf Trockengewichtsbasis;
- weniger als 5 % Fett auf Trockengewichtsbasis;
- einen Fluoridgehalt von 0,1 bis 200 mg/kg Hydrolysat auf Trockengewichtsbasis;
- einen TMAO(Trimethylamin-N-oxid)-Gehalt von 0,1 bis 200 mg N/100 g Hydrolysat auf Trockengewichtsbasis;
- einen TMA(Trimethylamin)-Gehalt von 0,1 bis 200 mg N/100 g Hydrolysat auf Trockengewichtsbasis;
- einen TVN-Gehalt (gesamter Gehalt an flüchtigem Stickstoff) von 0,1 bis 200 mg N/100 g Hydrolysat auf Trockengewichtsbasis;
- einen Natriumgehalt von 0,01 bis 4,0 g/100 g Hydrolysat auf Trockengewichtsbasis; und
- einen Calciumgehalt von 100 bis 25.000 mg/kg Hydrolysat auf Trockengewichtsbasis.

2. Nahrungsmittelprodukt nach Anspruch 1, wobei das Nahrungsmittelprodukt ein Nährstoffergänzungsmittel, vorzugsweise ein Nährstoffergänzungsmittel in Form eines Energieriegels, eines Mahlzeitenersatzriegels oder eines Getränks ist.

3. Nahrungsmittelprodukt nach Anspruch 1, wobei das Nahrungsmittelprodukt Nahrungsmittel ist, ausgewählt aus der Gruppe bestehend aus Getränken, Backwaren, Puddings, Milchprodukten, Konfekt, Snacks, gefrorenem Konfekt oder Neuheiten, zubereiteten gefrorenen Mahlzeiten, Süßigkeiten, Snackprodukten, Suppen, Aufstrichen, Soßen, Salatdressings, zubereiteten Fleischprodukten, Käse und Joghurt.

4. Nahrungsmittelprodukt nach Anspruch 3, wobei
- die Getränke aus der Gruppe ausgewählt sind, die aus Erfrischungsgetränken, Milch und anderen Milchgetränken und Diätgetränken besteht;
- das gefrorenen Konfekt oder Neuheiten aus der Gruppe ausgewählt sind, die aus Eiscreme und Milchshake besteht; und
- die Snackprodukte Chips sind.

5. Nahrungsmittelprodukt nach Anspruch 1, wobei das Nahrungsmittelprodukt ein Nahrungsergänzungsmittel ist.

6. Nahrungsmittelprodukt nach Anspruch 1, wobei das Nahrungsmittelprodukt ausgewählt ist aus der Gruppe bestehend aus Nahrungsmitteln, Getränken, funktionellen Nahrungsmitteln, Tierfutterrationen, pulverförmigen Proteinergänzungsmitteln, Mahlzeitenersatz, Getränkepulvern und Proteinshakepulvern.

7. Nahrungsmittelprodukt nach Anspruch 1, wobei das Nahrungsmittelprodukt ein Tierfutter, wie etwa ein für Begleittiere formuliertes Tierfutter oder ein für Haustiere formuliertes Tierfutter, ist.

8. Nahrungsmittelprodukt nach Anspruch 1 in Kombination mit unbedenklichen Hilfsstoffen und/oder Trägern, wobei das Nahrungsmittelprodukt eine Formulierung zum oralen Verzehr ist.

9. Nahrungsmittelprodukt nach Anspruch 8, wobei der Träger eine Flüssigkeit, ein Gel, eine Gelatinekapsel, eine Kapsel, ein Pulver, eine feste Tablette oder ein Tee ist.

10. Nahrungsmittelprodukt nach Anspruch 8, wobei der unbedenkliche Hilfsstoff und/oder Träger ausgewählt ist aus der Gruppe bestehend aus Pflanzenöl, Fischöl, Krillöl, Maltodextrin, Calciumcarbonat, Dicalciumphosphat, Tricalciumphosphat, mikrokristalliner Cellulose, Dextrose, Reismehl, Magnesiumstearat, Stearinsäure, Croscarmellose-Natrium, Natriumstärkeglycolat, Crospovidon, Saccharose, Pflanzengummis, Lactose, Methylcellulose, Povidon, Carboxymethylcellulose, Maisstärke und Mischungen davon; insbesondere Calciumcarbonat, Magnesiumstearat, Maltodextrin oder Mischungen davon.

11. Nahrungsmittelprodukt nach Anspruch 1 in Kombination mit Aromastoffen oder Süßungsmitteln, wobei das Nahrungsmittelprodukt eine Formulierung zur oralen Verabreichung ist.

12. Verwendung eines Krillproteinhydrolysats bei der Herstellung eines Nahrungsergänzungsmittels, Nährstoffergänzungsmittel, funktionellen Nahrungsmittels, Nahrungsmittels oder Getränks; wobei das Krillproteinhydrolysat Folgendes umfasst:
- einen Proteingehalt von mehr als 85 % auf Trockengewichtsbasis;
- weniger als 5 % Fett auf Trockengewichtsbasis;
- einen Fluoridgehalt von 0,1 bis 200 mg/kg Hydrolysat auf Trockengewichtsbasis;
- einen TMAO(Trimethylamin-N-oxid)-Gehalt von 0,1 bis 200 mg N/100 g Hydrolysat auf Trockengewichtsbasis;
- einen TMA(Trimethylamin)-Gehalt von 0,1 bis 200 mg N/100 g Hydrolysat auf Trockengewichtsbasis;
- einen TVN-Gehalt (gesamter Gehalt an flüchtigem Stickstoff) von 0,1 bis 200 mg N/100 g Hydrolysat auf Trockengewichtsbasis;
- einen Natriumgehalt von 0,01 bis 4,0 g/100 g Hydrolysat auf Trockengewichtsbasis; und
- einen Calciumgehalt von 100 bis 25.000 mg/kg Hydrolysat auf Trockengewichtsbasis.

13. Nahrungsmittelprodukt gemäß einem der Ansprüche 1-11 oder Verwendung gemäß Anspruch 12, wobei das Krillproteinhydrolysat einen Fluoridgehalt von 0,1 bis 30 mg/kg auf Trockengewichtsbasis aufweist.

14. Nahrungsmittelprodukt gemäß einem der Ansprüche 1-11 oder Verwendung gemäß Anspruch 12, wobei das Krillproteinhydrolysat ferner **dadurch gekennzeichnet ist, dass** es einen Proteingehalt von mehr als 89 % auf Trockengewichtsbasis und einen Fettgehalt von weniger als 2 % auf Trockengewichtsbasis aufweist.

15. Nahrungsmittelprodukt gemäß einem der Ansprüche 1-11 oder Verwendung gemäß Anspruch 12, wobei das Krillproteinhydrolysat **dadurch gekennzeichnet ist, dass** es einen Proteingehalt von mehr als 90 %, 91 %, 92 %, 93 %, 94 % oder 95 % auf Trockengewichtsbasis und/oder einen Fettgehalt von weniger als 3 %, 2 % oder 1 % auf Trockengewichtsbasis aufweist.

## Revendications

1. Produit alimentaire comprenant un hydrolysat de protéines de krill, l'hydrolysat de protéines de krill comprenant :
- une teneur en protéines de plus 85 % en poids sec ;
- moins de 5 % de matières grasses en poids sec ;
- une teneur en fluorure de 0,1 à 200 mg/kg d'hydrolysat en poids sec ;
- une teneur en TMAO (N-oxyde de triméthylamine) de 0,1 à 200 mg N/100 g d'hydrolysat en poids sec ;
- une teneur en TMA (triméthylamine) de 0,1 à 200 mg N/100 g d'hydrolysat en poids sec ;
- une teneur en TVN (azote volatil total) de 0,1 à 200 mg N/100 g d'hydrolysat en poids sec ;
- une teneur en sodium de 0,01 à 4,0 g/100 g d'hydrolysat en poids sec ; et
- une teneur en calcium de 100 à 25 000 mg/kg d'hydrolysat en poids sec.

2. Produit alimentaire de la revendication 1, dans lequel le produit alimentaire est un supplément nutritionnel, de préférence un supplément nutritionnel sous la forme de barre énergétique, de barre de substitut de repas ou d'une boisson.

3. Produit alimentaire de la revendication 1, dans lequel le produit alimentaire est des aliments choisis dans le groupe constitué par des boissons, des produits de boulangerie, des puddings, des produits laitiers, des confiseries, des aliments de collation, des confiseries ou sucreries congelées, des repas congelés préparés, des bonbons, des produits de collation, des soupes, des pâtes à tartiner, des sauces, des vinaigrettes, des produits carnés préparés, du fromage et du yaourt.

4. Produit alimentaire de la revendication 3, dans lequel
- les boissons sont choisies dans le groupe constitué de boissons sans alcool, de lait et d'autres boissons laitières, et de boissons diététiques ;
- les confiseries ou sucreries surgelées sont choisies dans le groupe constitué de crème glacée et de lait frappé ; et
- les produits de collation sont des chips.

5. Produit alimentaire de la revendication 1, dans lequel le produit alimentaire est un supplément alimentaire.

6. Produit alimentaire de la revendication 1, dans lequel le produit alimentaire est choisi dans le groupe constitué d'aliments, de boissons, d'aliments fonctionnels, de rations alimentaires pour animaux, de suppléments protéinés en poudre, de substituts de repas, de poudres de boisson et de poudres de frappé protéiné.

7. Produit alimentaire de la revendication 1, dans lequel le produit alimentaire est un aliment pour animaux, tel qu'un aliment pour animaux formulé pour des animaux de compagnie ou un aliment pour animaux formulé pour des animaux domestiques.

8. Produit alimentaire de la revendication 1, en combinaison avec des excipients et/ou des supports acceptables, dans lequel le produit alimentaire est une formulation pour la consommation orale.

9. Produit alimentaire de la revendication 8, dans lequel le support est un liquide, un gel, une capsule de gel, une capsule, une poudre, un comprimé solide ou du thé.

10. Produit alimentaire de la revendication 8, dans lequel l'excipient et/ou le support acceptable est choisi dans le groupe constitué par de l'huile végétale, de l'huile de poisson, de l'huile de krill, de la maltodextrine, du carbonate de calcium, du phosphate dicalcique, du phosphate tricalcique, de la cellulose microcristalline, du dextrose, de la farine de riz, du stéarate de magnésium, de l'acide stéarique, de la croscarmellose sodique, du glycolate d'amidon sodique, de la crospovidone, du saccharose, des gommes végétales, du lactose, de la méthylcellulose, de la povidone, de la carboxyméthylcellulose, de l'amidon de maïs et leurs mélanges ; en particulier du carbonate de calcium, du stéarate de magnésium, de la maltodextrine ou leurs mélanges.

11. Produit alimentaire de la revendication 1, en combinaison avec des agents aromatisants ou des édulcorants, dans lequel le produit alimentaire est une formulation pour une administration orale.

12. Utilisation d'un hydrolysat de protéines de krill dans la préparation d'un complément alimentaire, d'un complément nutritionnel, d'un aliment fonctionnel, d'un aliment ou d'une boisson ; l'hydrolysat de protéines de krill comprenant :
- une teneur en protéines de plus 85 % en poids sec ;
- moins de 5 % de matières grasses en poids sec ;
- une teneur en fluorure de 0,1 à 200 mg/kg d'hydrolysat en poids sec ;
- une teneur en TMAO (N-oxyde de triméthylamine) de 0,1 à 200 mg N/100 g d'hydrolysat en poids sec ;
- une teneur en TMA (triméthylamine) de 0,1 à 200 mg N/100 g d'hydrolysat en poids sec ;
- une teneur en TVN (azote volatil total) de 0,1 à 200 mg N/100 g d'hydrolysat en poids sec ;
- une teneur en sodium de 0,01 à 4,0 g/100 g d'hydrolysat en poids sec ; et
- une teneur en calcium de 100 à 25 000 mg/kg d'hydrolysat en poids sec.

13. Produit alimentaire selon l'une quelconque des revendications 1 à 11 ou utilisation selon la revendication 12, dans lesquels l'hydrolysat de protéine de krill présente une teneur en fluorure de 0,1 à 30 mg/kg en poids sec.

14. Produit alimentaire selon l'une quelconque des revendications 1 à 11 ou utilisation selon la revendication 12, dans lesquels l'hydrolysat de protéine de krill est en outre **caractérisé en ce qu'**il présente une teneur en protéines de plus de 89 % en poids sec et une teneur en matières grasses de moins de 2 % en poids sec.

15. Produit alimentaire selon l'une quelconque des revendications 1 à 11 ou utilisation selon la revendication 12, dans lesquels l'hydrolysat de protéine de krill est **caractérisé en ce qu'**il présente une teneur en protéines de plus de 90 %, 91 %, 92 %, 93 %, 94 % ou 95 % en poids sec et/ou une teneur en matières grasses de moins de 3 %, 2 % ou 1 % en poids sec.
